Europäisches Patentamt

European Patent Office

Office européen des brevets

(19)

(11) Numéro de publication : **0 037 137**
**B1**

(12) **FASCICULE DE BREVET EUROPÉEN**

(45) Date de publication du fascicule du brevet : •
02.05.84

(21) Numéro de dépôt : **81200273.1**

(22) Date de dépôt : **12.03.81**

(51) Int. Cl.³ : **B 01 J 23/96**// C07C31/18,
C07C31/26, C07C29/00

(54) **Procédé de réactivation d'un catalyseur, à base de métaux du groupe du platine, pour l'hydrogénation des sucres.**

(30) Priorité : **18.03.80 BE 199835**

(43) Date de publication de la demande :
**07.10.81 Bulletin 81/40**

(45) Mention de la délivrance du brevet :
**02.05.84 Bulletin 84/18**

(84) Etats contractants désignés :
**DE FR GB NL**

(56) Documents cités :
**BE-A- 837 201**
**DE-B- 1 053 121**
**FR-A- 1 083 606**
**GB-A- 1 055 672**
**GB-A- 1 156 398**
**US-A- 2 868 847**
**US-A- 3 248 338**
**US-A- 3 684 740**
**US-A- 3 963 789**
**US-A- 3 966 636**

(73) Titulaire : **Université de Liège**
**7, Place du XX Août**
**B-4000 Liège (BE)**

(72) Inventeur : **Maisin, Armand**
**23 rue Fernand Piette**
**Bas-Oha (Huy) (BE)**
Inventeur : **Lefevre, André**
**39, Quai des Ardennes**
**B-4000 Liège (BE)**
Inventeur : **Germain, Albert**
**4, rue Simonon**
**B-4000 Liège (BE)**
Inventeur : **Wauters, Marc**
**18, rue Discry**
**Flémalle (BE)**

(74) Mandataire : **Pirson, Jean et al**
**c/o Bureau Gevers 7, rue de Livourne, Boîte no 1**
**B-1050 Bruxelles (BE)**

Jouve, 18, rue St-Denis, 75001 Paris, France

## Procédé de réactivation d'un catalyseur, à base de métaux du groupe du platine, pour l'hydrogénation des sucres

La présente invention a pour objet un procédé de réactivation d'un catalyseur, pour l'hydrogénation des sucres, à base d'au moins un des six métaux du groupe du platine, à savoir, ruthénium, rhodium, palladium, osmium, iridium et platine, et/ou d'au moins un oxyde desdits métaux.

L'utilisation de ces six métaux ou de leurs oxydes comme catalyseurs d'hydrogénation des sucres en alcools correspondants est connue comme en témoignent notamment les brevets U.S. 2.868.847 et 2.555.856 ainsi que le brevet belge 837.201. En général, le catalyseur est utilisé sous la forme d'une combinaison catalyseur-support, le catalyseur représentant de 0,1 à environ 10 % du poids total de la combinaison. Le catalyseur peut être constitué soit par un seul des métaux susdits, soit par un mélange de deux ou plusieurs de ces métaux à l'état élémentaire, ou soit par un seul, soit par un mélange d'oxydes d'un ou plusieurs de ces métaux, ou encore par un mélange d'un ou plusieurs de ces métaux et d'un ou plusieurs oxydes de ces métaux. Le support est constitué soit par une matière inerte telle que du carbone, de l'alumine, de l'argile, du kieselguhr, un gel synthétique, des diatomées et autres substances analogues, soit par une matière non inerte, telle qu'une zéolithe.

Ces catalyseurs sont évidemment coûteux et il est essentiel de réduire au maximum les pertes de catalyseur au cours du processus d'hydrogénation. Comme c'est généralement le cas pour les systèmes catalytiques, l'activité de ces catalyseurs diminue progressivement et il est nécessaire, après un certain temps d'utilisation, de réactiver le catalyseur ou de le remplacer. Le remplacement est évidemment une solution extrêmement coûteuse qui prohiberait l'utilisation de catalyseurs du type défini ci-avant dans tout processus d'hydrogénation industriel. Pour rendre ces catalyseurs économiquement utilisables, il faut donc les réactiver.

On connaît, suivant le brevet U.S.A. N° 3.963.789, un procédé de réactivation de catalyseur épuisé suivant lequel on traite le catalyseur et son support par lavage à l'acide. L'utilisation d'un acide présente l'inconvénient de provoquer des pertes par dissolution du support et des baisses d'activité par altération de la structure cristalline du support si celui-ci n'est pas inerte. De plus, l'utilisation d'un acide nécessite l'emploi de matériaux spéciaux pour résister à la corrosion et malgré cela, on ne pourrait pas envisager ce traitement acide, dans une colonne d'hydrogénation à lit fixe de catalyseur, pour la réactivation du catalyseur épuisé, car la plus petite modification de la structure physique du catalyseur et de son support peut conduire à des bouchages de la colonne, ce qui implique que la réactivation du catalyseur doit être conduite dans un appareillage distinct de celui utilisé pour l'hydrogénation, ce qui augmente les coûts de l'installation et présente aussi comme autre inconvénient d'entraîner de nombreuses manipulations du catalyseur, telles que filtration, transvasement, qui entraînent des pertes non négligeables.

On connaît aussi, suivant le brevet USA N° 3.966.636, un procédé suivant lequel on réactive le catalyseur épuisé, utilisé pour l'hydrogénation de composés organiques ayant au moins une liaison oléfinique, en le mettant successivement en contact, en l'absence du solvant, avec un gaz contenant, en volume, de 1 % à 15 % d'hydrogène pendant 1/2 heure à 2 heures à une température comprise entre 400 et 600 °C, puis avec un gaz contenant, en volume, de 1 à 10 % d'oxygène pendant 1 heure à 3 heures à une température comprise entre 350 °C et 450 °C et enfin avec un gaz contenant, en volume, de 1 à 100 % d'hydrogène à une température s'échelonnant de 150 °C à 600 °C pendant 1/2 heure à 1 heure. Outre qu'un travail à sec et à de si hautes températures présente également l'inconvénient de nécessiter que la réactivation soit conduite dans un appareillage distinct du réacteur d'hydrogénation, ce procédé ne convient pas pour la réactivation des catalyseurs épuisés ayant servi à l'hydrogénation des sucres. En effet, la dernière étape de réactivation par hydrogénation fournirait un catalyseur trop réduit qui aurait perdu la majeure partie de son activité d'hydrogénation des sucres, aux conditions modérées de température et de pression utilisées pour l'hydrogénation desdits sucres.

On connaît encore, suivant le brevet britannique N° 1.156.398, un procédé pour la régénération d'un catalyseur métallique noble du groupe VIII, sur un support comprenant de l'alumine, qui a été utilisé pour la préparation d'un ester organique insaturé par réaction d'un composé organique oléfiniquement insaturé avec un acide carboxylique organique en présence d'oxygène, ce procédé comprenant une mise en contact du catalyseur avec de l'oxygène pour enlever tous les résidus organiques déposés sur le catalyseur, suivie d'une mise en contact de ce dernier avec de l'hydrogène pour réduire ce catalyseur à son état métallique. Ce procédé prévoit que la mise en contact du catalyseur avec l'oxygène s'effectue, pendant 2 à 6 heures, à une température comprise entre 150 et 800 °C, tandis que sa mise en contact avec l'hydrogène s'effectue, pendant 1/2 à 2 heures, à une température comprise entre 50 et 500 °C. Ledit procédé prévoit également le lavage, notamment avec de l'eau, du catalyseur avant son traitement à l'oxygène et avant son traitement à l'hydrogène, ainsi qu'une mise en contact du catalyseur avec un gaz inerte, d'une part, après la mise en contact du catalyseur avec l'oxygène et avant sa mise en contact avec l'hydrogène et, d'autre part, après sa mise en contact avec l'hydrogène.

Tout comme le procédé suivant le brevet USA N° 3.966.636, ce procédé présente l'inconvénient, vu les hautes températures mises en œuvre, de

2

nécessiter que la réactivation soit conduite dans un appareillage distinct du réacteur d'hydrogénation et de ne pas convenir pour la réactivation des catalyseurs épuisés ayant servi à l'hydrogénation des sucres, du fait que la dernière étape de réactivation par hydrogénation fournirait un catalyseur trop réduit qui aurait perdu une grande partie de son activité d'hydrogénation des sucres, aux conditions modérées de température et de pression utilisées pour cette hydrogénation des sucres.

On connaît enfin, suivant le brevet USA N° 3.684.740, un procédé suivant lequel on réactive le catalyseur épuisé, utilisé pour l'hydrogénation sélective d'hydrocarbones acétyléniques dans un courant riche en diène hydrocarboné, en présence d'hydrogène, en éliminant par combustion à partir du catalyseur le matériau carboné, en refroidissant ledit catalyseur et en le lavant à l'eau distillée. L'élimination par combustion s'effectue en chauffant le catalyseur avec de la vapeur jusqu'à une température de 343 °C à 454 °C et en le soumettant ensuite à un courant d'air et de vapeur qui porte sa température entre 426 °C et 537 °C.

Ce procédé présente également l'inconvénient de ne pouvoir être utilisé dans une colonne d'hydrogénation des sucres, pour la réactivation du catalyseur épuisé, du fait des hautes températures mises en œuvre.

La présente invention a pour but de remédier aux inconvénients précités et de procurer un procédé de réactivation des catalyseurs, à base d'un ou plusieurs des six métaux du groupe du platine, épuisés après avoir servi à l'hydrogénation de sucres en alcools correspondants, ce procédé présentant l'avantage de permettre la réactivation dans des conditions de réaction modérées, d'éviter des pertes de catalyseur et l'utilisation d'un appareillage coûteux distinct du réacteur d'hydrogénation, ledit procédé de réactivation selon l'invention pouvant en effet être appliqué directement dans le réacteur d'hydrogénation, avec pour avantage supplémentaire de ne pas nécessiter l'utilisation d'un matériau spécial anticorrosion pour la construction dudit réacteur comme c'est le cas dans la régénération par un acide.

Une des conséquences les plus avantageuses, qui découle du procédé suivant l'invention, est de rendre économiquement possible l'utilisation de catalyseurs à base d'un des métaux du groupe du platine pour l'hydrogénation continue des sucres sur un lit fixe de catalyseur. Jusqu'à présent, le catalyseur utilisé en lit fixe devait être remplacé lorsqu'il était épuisé (cf. Haidegger — Die Stärke, 1977, (12), 430-435) ce qui, vu les pertes encourues, prohibait l'usage de catalyseurs coûteux malgré leurs avantages bien connus sur les catalyseurs plus courants, tels que ceux à base de nickel.

A cet effet, le procédé suivant l'invention consiste à laver le catalyseur et son support à l'eau et à traiter ensuite ces derniers, jusqu'à réactivation complète du catalyseur à l'aide d'un gaz contenant, en volume, 1 à 100 % d'oxygène, le traitement du catalyseur et de son support par ce gaz s'effectuant à une température comprise entre 80 et 150 °C et à une pression comprise entre 5 et 10 bars.

D'autres détails et particularités de l'invention ressortiront de la description du procédé donnée ci-après à titre d'exemple non limitatif et illustré au graphique annexé.

Suivant l'invention, le catalyseur épuisé et son support sont lavés par un faible débit d'eau et traités, jusqu'à réactivation complète, par un gaz contenant, en volume, 1 à 100 % d'oxygène, à une pression comprise entre 5 et 10 bars et à une température comprise entre 80 et 150 °C.

La durée de l'opération de réactivation est, naturellement, fonction du débit de gaz qui est compris entre 400 et 6 000 l N/litre de catalyseur-support par heure. Par exemple pour un débit de 1 000 à 2 000 lN (litre normal)/litre de catalyseur-support par heure, l'opération est déjà terminée après 3 heures.

Pour éviter toute réaction secondaire, il est souhaitable d'éliminer toute trace de sucre et d'hydrogène par un lavage à l'eau et un balayage par un gaz inerte, tel que l'azote, l'argon, etc..., du catalyseur épuisé avant l'opération de réactivation.

De même, avant de réutiliser le catalyseur réactivé, il est souhaitable d'éliminer toute trace d'oxygène par balayage par un gaz inerte.

Ces opérations de lavage et de balayage seront, de préférence, conduites à une température de 50 à 150 °C et à une pression de 5 à 100 bars avec un débit d'eau de 0,5 à 10 litres/litre de catalyseur-support par heure et un débit de gaz de 300 à 6 000 lN/l de catalyseur-support par heure et ceci afin de rendre ces opérations aussi rapides que possible.

Un essai de réactivation d'un catalyseur constitué de ruthénium supporté par de la $\gamma$ Alumine, à raison de 2 % de ruthénium par rapport au poids total de la combinaison ruthénium-support et utilisé pour l'hydrogénation en lit fixe du maltose en maltitol a permis d'établir le graphique annexé. Comme il ressort de ce graphique, la réactivation dans les conditions énumérées ci-dessous, après lavage à l'eau et balayage à l'argon, fait passer l'activité du catalyseur épuisé, mesurée en % de conversion du sucre en polyol, de 60 à 100 après une durée de 16 h 40.

Conditions de réactivation

pression $O_2$ = 8 bars
débit $O_2$ > 1 000 lN/l de catalyseur-support par heure
température : 100 °C
débit $H_2O$ = 0,6 l/l de catalyseur-support par heure
poids du catalyseur et son support = 30,2 grammes.

Les quatre exemples concrets donnés ci-après permettent d'illustrer davantage le procédé suivant l'invention, appliqué à l'hydrogénation de sucres en polyols correspondants sur lit fixe de catalyseur, et de mettre en évidence les avantages qu'il procure.

Exemple 1 — Hydrogénation du maltose en maltitol

Dans une colonne thermostatisée à 95 °C, on a placé 30,2 g d'un catalyseur ruthénium-alumine γ à 2 % de ruthénium. La colonne est alimentée en continu, au sommet, par une solution aqueuse à 30 % en poids de maltose à pH 4,3 coulant à un débit de 0,83 l/l de catalyseur-support par heure et par un courant gazeux d'hydrogène à une pression de 70 bars et à un débit de 830 IN/l de catalyseur-support par heure. La solution de maltitol est recueillie en continu à la base de la colonne. Toutes conditions étant maintenues constantes, le taux de conversion du maltose, de 100 % au départ, diminue progressivement au cours du temps. Après 55 heures, lorsque ce taux de conversion atteint 79,5 %, on procède à la réactivation du catalyseur. La solution aqueuse de maltose est remplacée par de l'eau à une température de 100 °C coulant à un débit de 0,6 l/l de catalyseur-support par heure pendant toute la suite des opérations jusqu'à la remise en service du catalyseur réactivé. Simultanément, l'alimentation en hydrogène est interrompue et remplacée par de l'argon à une pression de 70 bars et à un débit de 830 IN/l de catalyseur-support par heure. Lorsque toutes traces de sucre et d'hydrogène ont disparu, c'est-à-dire après 1,5 heure, l'alimentation en gaz inerte est interrompue et remplacée par de l'air (ou oxygène pur) à une pression de 8 bars et à un débit de 1 100 IN/l catalyseur-support par heure. La température est maintenue à 100 °C.

Après 8 heures, la régénération est terminée, l'alimentation en oxygène est interrompue, l'installation est purgée de son oxygène qui est remplacé par de l'argon à une pression de 70 bars et à un débit de 830 IN/l de catalyseur-support par heure. Lorsque toute trace d'oxygène a disparu, c'est-à-dire après 1 heure, on peut reprendre l'hydrogénation, ce qui se fait très simplement en remplaçant l'eau par la solution de maltose et l'argon par l'hydrogène.

Exemple 2 — Hydrogénation du fructose en mannitol et sorbitol.

La même colonne thermostatisée à 92 °C (voir exemple 1) a été remplie par 60 g d'un catalyseur contenant 2 % de ruthénium déposé sur alumine γ. La concentration d'alimentation est de 100 g/l de fructose, la pression de 30 bars. Le débit de solution est fixé à 1,4 l/l catalyseur-support par heure alors que le débit d'hydrogène est de 420 IN/l catalyseur-support par heure. En maintenant toutes les variables constantes, on observe que la conversion totale (mannitol + sorbitol) passe de 98,4 % à 97,6 % en 82 heures de fonctionnement continu, moment auquel on procède à la réactivation. La solution aqueuse de fructose est remplacée par de l'eau à une température de 110 °C coulant au débit de 0,83 l/l de catalyseur-support par heure pendant toute la réactivation jusqu'à la remise en service du catalyseur réactivé. Simultanément, l'alimentation en hydrogène est remplacée par de l'argon à une pression de 30 bars et à un débit de 625 IN/l de catalyseur-support par heure. Après 1 heure, l'argon est remplacé par de l'oxygène pur à une pression de 6 bars et à un débit de 420 IN/l de catalyseur-suppport par heure. La température est maintenue à 110 °C. Après 7 heures la régénération est terminée, l'oxygène interrompu est purgé et remplacé par de l'argon à une pression de 30 bars et à un débit de 625 IN/l de catalyseur-support par heure. Après 1 heure on reprend l'hydrogénation en remplaçant l'eau par la solution de fructose et l'argon par l'hydrogène.

Exemple 3 — Hydrogénation du glucose en sorbitol.

La même colonne (voir exemples 1 et 2) thermostatisée à 105 °C a été remplie par 9,3 g d'un catalyseur contenant 2 % de ruthénium déposé sur charbon. La concentration de l'alimentation est de 300 g/l de glucose et la pression de 45 bars. Le débit de la solution est fixé à 8,7 l/l de catalyseur-support par heure alors que le débit d'hydrogène est de 4 600 IN/l de catalyseur-support par heure. En maintenant toutes les variables constantes, on observe que la conversion en sorbitol passe de 94 % à 78,1 % en 64 heures de fonctionnement continu, moment auquel on procède à la réactivation. La solution aqueuse de glucose est remplacée par de l'eau à une température de 105 °C coulant au débit de 7,3 l/l de catalyseur-support par heure. Simultanément, l'alimentation en hydrogène est remplacée par de l'argon à une pression de 8 bars et à un débit de 5 500 IN/l de catalyseur-support par heure. Après 1/2 heure, l'argon est remplacé par de l'oxygène pur à une pression de 8 bars et à un débit de 3 200 IN/l de catalyseur-support par heure, tandis que le débit d'eau ramené à 1,8 l/l de catalyseur-support par heure. La température est élevée à 115 °C. Après 9 heures la régénération est terminée, l'oxygène interrompu est purgé et remplacé par de l'argon à une pression de 45 bars et à un débit de 5 500 IN/l de catalyseur-support par heure. Après 1 heure, on reprend l'hydrogénation en remplaçant l'eau par la solution de glucose au débit de 8,7 l/l de catalyseur-support par heure et l'argon par l'hydrogène à un débit de 4 600 IN/l de catalyseur-support par heure.

Exemple 4 — Hydrogénation du saccharose en sorbitol et mannitol.

La même colonne thermostatisée à 106 °C (voir exemples 1 à 3) a été remplie par 4,03 grammes d'un catalyseur contenant 2 % de ruthénium déposé sur alumine γ. La concentration de l'alimentation est de 150 grammes de saccharose par litre, la pression d'hydrogène est de 60 bars. Le débit de solution est fixé à 18 l/l de catalyseur-support par heure alors que le débit d'hydrogène est de 8 700 IN/l de catalyseur-support par heure. En maintenant toutes les variables constantes, on observe que la conversion totale passe de 33,5 % à 30,8 % (le rapport mannitol/sorbitol est égal à 0,69) en 78,5 heures de fonctionnement continu, moment auquel on procède à la réactivation. La solution aqueuse de saccharose est remplacée par de l'eau à une température de 106 °C coulant au débit de 10 l/l de catalyseur-support par heure. Simultanément, l'alimentation en hydrogène est remplacée par de l'argon à une pression de 60 bars et à un débit de 5 500 IN/l de catalyseur-support par heure. Après 45 minutes, l'argon est remplacé par de l'oxygène pur à une pression de 10 bars et à un débit de 5 000 IN/l de catalyseur-support par heure, tandis que le débit d'eau est ramené à 1,5 l/l de catalyseur-support par heure. La température est maintenue à 106 °C. Après 12 heures, on arrête la régénération, l'oxygène est interrompu et purgé et remplacé par de l'argon à une pression de 60 bars et à un débit de 5 500 IN/l de catalyseur-support par heure. Après 45 minutes, on reprend l'hydrogénation en remplaçant l'eau par la solution de saccharose au débit de 18 l/l de catalyseur-support par heure et l'argon par l'hydrogène à un débit de 8 700 IN/l de catalyseur-support par heure.

Exemple 5 — Hydrogénation du glucose en sorbitol.

La même colonne thermostatisée à 86,5 °C (voir exemples 1 à 4) a été remplie par 10,01 g d'un catalyseur contenant 2 % de ruthénium déposé par alumine γ. La concentration de l'alimentation est de 300 grammes de glucose par litre et la pression d'hydrogène de 40 bars. Le débit de solution est fixé à 6,5 l/l de catalyseur-support par heure, alors que le débit d'hydrogène est de 10 000 IN/l de catalyseur-support par heure. En maintenant toutes les variables constantes, on observe que la conversion en sorbitol passe de 18,6 % à 6,9 % en 375 heures de fonctionnement continu. On décide alors de procéder à la réactivation. La solution aqueuse de glucose est remplacée par de l'eau à une température de 86,5 °C coulant à un débit de 6 l/l de catalyseur-support par heure. Simultanément, l'alimentation en hydrogène est remplacée par de l'argon à une pression de 5 bars et à un débit de 6 000 IN/l de catalyseur-support par heure. Après 2 heures 30, l'argon est remplacé par de l'air (réactivation par un flux d'oxygène dilué) à une pression de 10 bars et à un débit de 6 000 IN/l de catalyseur-support par heure, tandis que le débit d'eau est ramené à 2 l/l catalyseur-support par heure. La température est élevée à 150 °C. Après 4,5 heures de régénération, le débit d'air est interrompu, purgé et remplacé par de l'argon à une pression de 10 bars et un débit de 6 000 IN/l de catalyseur-support par heure. Après 1 heure, on reprend l'hydrogénation en remplaçant l'eau par la solution de glucose au débit de 6,5 l/l de catalyseur-support par heure et l'argon par de l'hydrogène à un débit de 10 000 IN/l de catalyseur-support par heure.

Les exemples donnés ci-avant le sont uniquement à titre indicatif. Il est bien entendu que l'invention peut être appliquée pour n'importe quel catalyseur tel que défini ci-avant ayant servi à l'hydrogénation de n'importe quel sucre, maltose, glucose, fructose, saccharose, etc... en alcools correspondants.

De même, s'il est préféré de travailler en lit fixe, le procédé de réactivation selon l'invention étant particulièrement avantageux dans ce cas, ledit procédé peut également s'appliquer à la réactivation d'un catalyseur épuisé dans un autoclave avec agitation. Dans ce dernier cas, le procédé suivant l'invention offre, par rapport aux procédés connus, l'avantage de respecter la structure du catalyseur et d'éviter les pertes par attaque du support.

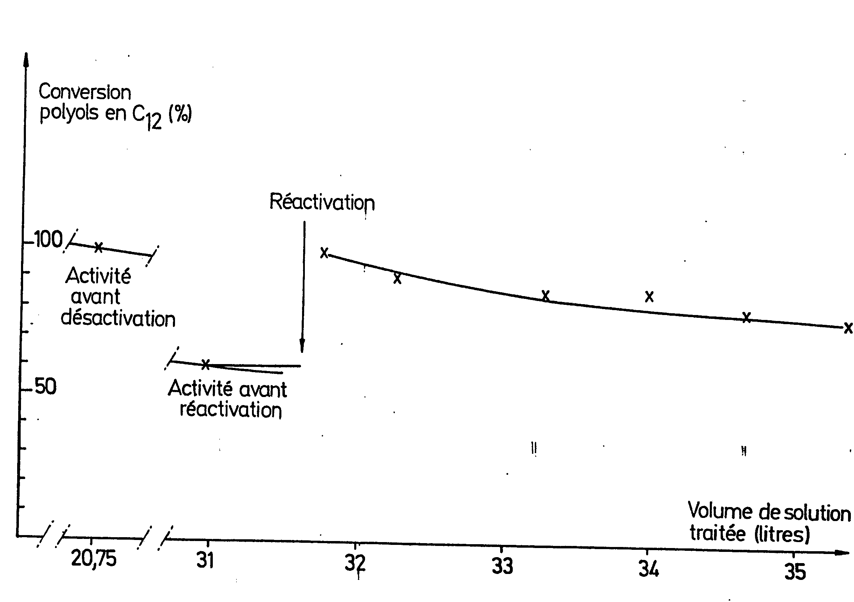

## Revendications

1. Procédé de réactivation d'un catalyseur, à base d'au moins un métal du groupe du platine et/ou d'au moins un oxyde desdits métaux, pour l'hydrogénation des sucres, caractérisé en ce qu'il consiste à laver le catalyseur et son support à l'eau et à traiter ensuite ces derniers, jusqu'à réactivation complète du catalyseur, à l'aide d'un gaz contenant, en volume, 1 à 100 % d'oxygène, le traitement du catalyseur et de son support par ce gaz s'effectuant à une température comprise entre 80 et 150 °C et à une pression comprise entre 5 et 10 bars.

2. Procédé suivant la revendication 1, caractérisé en ce que le catalyseur et son support sont soumis, préalablement à leur traitement à l'aide du gaz précité, à un balayage par un gaz inerte, tel que l'azote, l'argon.

3. Procédé suivant l'une ou l'autre des revendications 1 et 2, caractérisé en ce que le catalyseur et son support sont soumis, après leur traitement à l'aide du gaz contenant de l'oxygène, à un balayage par un gaz inerte.

4. Procédé suivant la revendication 3, caractérisé en ce que le lavage à l'eau du catalyseur et de son support est poursuivi pendant que ceux-ci sont traités par le gaz contenant de l'oxygène et soumis aux balayages susdits.

5. Procédé suivant l'une quelconque des revendications 1 à 4, caractérisé en ce que le lavage du catalyseur et de son support s'effectue à une température comprise entre 50 et 150 °C, avec un débit d'eau de 0,5 à 10 litres/litre de catalyseur-support par heure et à une pression comprise entre 5 et 100 bars.

6. Procédé suivant l'une quelconque des revendications 1 à 5, caractérisé en ce que le traitement précité s'effectue avec un débit de gaz contenant de l'oxygène compris entre 400 et 6 000 IN/litre de catalyseur-support par heure.

7. Procédé suivant l'une quelconque des revendications 3 à 6, caractérisé en ce que le balayage par le gaz inerte susdit s'effectue à une température comprise entre 50 et 150 °C, avec un débit compris entre 300 et 6 000 lN (litre normal)/litre de catalyseur-support par heure et à une pression comprise entre 5 et 100 bars.

8. Procédé suivant l'une quelconque des revendications 1 à 7, caractérisé en ce que les opérations de réactivation s'effectuent dans la colonne d'hydrogénation des sucres.

## Claims

1. Process for the reactivating of a catalyst, on the basis of at least one metal from the platinum group and/or at least one oxide of said metals, for the hydrogenation of the sugars, characterized in that it comprises washing the catalyst and the support thereof with water, and treating these latter ones thereafter until the complete reactivation of the catalyst, by means of a gas containing 1 to 100 % by volume of oxygen, the treatment of the catalyst and of the support thereof with this gas being performed at a temperature lying between 80 and 150 °C and under a pressure lying between 5 and 10 bars.

2. Process according to the claim 1, characterized in that the catalyst and the support thereof are subjected previous to the treatment thereof by means of said gas, to a scavenging with an inert gas such as nitrogen, argon.

3. Process according to either one of the claims 1 and 2, characterized in that the catalyst and the support thereof are subjected after the treatment thereof by means of the gas containing oxygen, to a scavenging with an inert gas.

4. Process according to the claim 3, characterized in that the washing of the catalyst and the support thereof with water is continued while these latter ones are treated with the gas containing oxygen and subjected to said scavengings.

5. Process according to any one of the claims 1 to 4, characterized in that the washing of the catalyst and the support thereof is performed at a temperature lying between 50 and 150 °C, with a water flow rate from 0,5 to 10 litres/litre of catalyst-support per hour and under a pressure lying between 5 and 100 bars.

6. Process according to any one of the claims 1 to 5, characterized in that said treatment is performed with a flow rate of the gas containing oxygen lying between 400 and 6 000 lN/litre of catalyst-support per hour.

7. Process according to any one of the claims 3 to 6, characterized in that the scavenging with said inert gas is performed at a temperature lying between 50 and 150 °C, with a flow rate lying between 300 and 6 000 lN (normal litre)/litre of catalyst-support per hour and under a pressure lying between 5 and 100 bars.

8. Process according to any one of the claims 1 to 7, characterized in that the reactivating operations are performed inside the column for the hydrogenation of the sugars.

## Ansprüche

1. Verfahren zum Reaktivieren eines Katalysators auf Basis wenigstens eines Metalls der Platingruppe und/oder wenigstens eines Oxyds dieser Metalle zur Hydrierung von Zucker, dadurch gekennzeichnet, daß der Katalysator und sein Träger mit Wasser gewaschen werden und anschließend beide bis zur vollständigen Reaktivierung des Katalysators mit einem 1 bis 100 Vol.-% Sauerstoff enthaltenden Gas behandelt werden, wobei die Behandlung des Katalysators und seines Trägers mit diesem Gas bei einer Temperatur zwischen 80 und 150 °C und einem Druck zwischen 5 und 10 Bar durchgeführt wird.

2. Verfahren nach Anspruch 1, dadurch gekennzeichnet, daß der Katalysator und sein Träger vor der vorgenannten Gasbehandlung mit einem Inertgas wie Stickstoff, Argon gespült werden.

3. Verfahren nach Anspruch 1 oder 2, dadurch gekennzeichnet, daß der Katalysator und sein Träger nach der Behandlung mit dem Sauerstoff enthaltenden Gas mit einem Inertgas gespült werden.

4. Verfahren nach Anspruch 3, dadurch gekennzeichnet, daß das Waschen des Katalysators und seines Trägers mit Wasser fortgesetzt wird, während diese mit dem Sauerstoff enthaltenden Gas behandelt und der vorgenannten Spülung ausgesetzt werden.

5. Verfahren nach irgendeinem der Ansprüche 1 bis 4, dadurch gekennzeichnet, daß das Waschen des Katalysators und seines Trägers bei einer Temperatur zwischen 50 und 150 °C, einer Wassermenge von 0,5 bis 10 Litern/Liter Katalysator-Träger pro Stunde und einem Druck zwischen 5 und 100 Bar durchgeführt wird.

6. Verfahren nach irgendeinem der Ansprüche 1 bis 5, dadurch gekennzeichnet, daß die vorgenannte Behandlung mit einer Menge zwischen 400 und 600 lN (Normal-liter)/Liter Katalysator-Träger pro Stunde durchgeführt wird.

7. Verfahren nach irgendeinem der Ansprüche 3 bis 6, dadurch gekennzeichnet, daß das vorgenannte Spülen mit dem Inertgas bei einer Temperatur zwischen 50 und 150 °C, einer Menge zwischen 300 und 6 000 lN (Normal-liter)/Liter Katalysator-Träger pro Stunde und einem Druck zwischen 5 und 100 Bar durchgeführt wird.

8. Verfahren nach irgendeinem der Ansprüche 1 bis 7, dadurch gekennzeichnet, daß das Reaktivierungsverfahren in der Zucker-Hydrierungskolonne durchgeführt wird.

Conversion polyols en $C_{12}$ (%)

Réactivation

100

Activité avant désactivation

Activité avant réactivation

50

Volume de solution traitée (litres)

20,75   31   32   33   34   35